# DEMANDE DE BREVET EUROPEEN

(11) **EP 1 600 150 A1**
(43) Date de publication de la demande: **30.11.2005**
(21) Numéro de dépôt: 05291146.8
(22) Date de dépôt: 27.05.2005
(51) Int. Cl.: A61K 7/135

(54) **Composition pour le traitement de matières kératiniques comprenant un composé polycarboxylique particulier et un polymère épaississant et procédés la mettant en oeuvre**

(30) Priorité: 28.05.2004 FR 0405843
(71) Demandeur: L'OREAL, 75008 Paris (FR)
(72) Inventeur: Legrand, Frédéric, 92400 Courbevoie (FR); Cottard, François, 92400 Courbevoie (FR)
(74) Mandataire: Wattremez, Catherine

(57) **Abrégé**

La présente invention a pour objet une composition destinée au traitement des fibres kératiniques humaines, comprenant un composé portant quatre fonctions acide carboxylique, sous forme acide ou sous forme de sel, ledit composé étant associé à au moins un polymère épaississant particulier.

Elle concerne de plus des procédés de déformation, décoloration, coloration de fibres kératiniques humaines la mettant en oeuvre.

## Description

La présente invention a pour objet une composition destinée au traitement des matières kératiniques humaines, comprenant un composé portant quatre fonctions acide carboxylique, sous forme acide ou sous forme de sel, ledit composé étant associé à au moins un polymère épaississant particulier. Elle concerne de plus des procédés traitement des matières kératiniques consistant à appliquer ladite composition. Elle a enfin pour objet des procédés de déformation, décoloration, coloration de fibres kératiniques humaines mettant en oeuvre la composition selon l'invention.

Depuis longtemps, on a cherché à modifier l'aspect, la couleur des fibres kératiniques humaines, et notamment des cheveux.

Ainsi, on peut mettre en oeuvre des procédés de déformation permanente réalisés habituellement en deux temps.

Plus spécialement, on effectue une première étape, durant laquelle les ponts disulfures présents dans les fibres sont ouverts, habituellement en présence d'un agent réducteur.

Avant, après, ou simultanément à la réduction de ces ponts disulfures, la fibre est mise en forme de la manière souhaitée (bigoudi, lissage).

Une fois cette première étape effectuée, il est nécessaire de mettre en oeuvre une étape durant laquelle les ponts disulfures sont reformés afin de stabiliser la forme obtenue. Cette étape de fixation de la forme est habituellement mise en oeuvre au moyen d'une composition comprenant un agent oxydant.

En ce qui concerne les procédés de décoloration des fibres, ces traitements sont effectués par oxydation du pigment "mélanine" aboutissant à la solubilisation et l'élimination partielle ou totale de ce pigment dans la fibre.

Dans ce but, on utilise des compositions décolorantes contenant au moins un réactif peroxygéné et au moins un agent alcalin comme activateur de ces composés peroxygénés. Ces compositions sont par la suite associées au moment de l'emploi à une composition aqueuse de peroxyde d'hydrogène.

Selon une autre possibilité, le but recherché par les procédés de décoloration peut être de décaper la fibre, en d'autre termes de la débarrasser des colorants artificiels présents.

Ce type de décoloration peut être fait soit en présence d'un agent oxydant, comme précédemment résumé, ou bien en présence d'un agent réducteur. L'agent actif mis en oeuvre est en fait choisi de manière classique par l'homme du métier, en fonction de la nature du colorant à éliminer.

Enfin, dans le domaine de la coloration, on connaît plusieurs types de procédés parmi lesquels figurent la coloration permanente (ou coloration d'oxydation) et la coloration directe (ou coloration semi-permanente) avec ou sans effet d'éclaircissement des fibres.

Dans le domaine de la coloration d'oxydation, les composés utilisés sont des précurseurs de colorants d'oxydation, plus particulièrement des bases d'oxydation éventuellement associées à un ou plusieurs coupleurs. Ces composés sont des substances incolores ou peu colorées qui, par un processus de condensation oxydative, en présence d'un agent oxydant, conduisent à des composés qui colorent les fibres.

Dans le domaine de la coloration semi-permanente, la composition appliquée sur les fibres comprend au moins des colorants directs, qui sont des composés colorants et colorés, et peut éventuellement aussi comprendre au moins un agent oxydant, si l'on souhaite obtenir un effet éclaircissant associé à la coloration.

Quel que soit le résultat souhaité, les compositions employées doivent satisfaire à des critères relativement contraignants du fait qu'elles sont appliquées directement sur les fibres et entrent en contact avec la peau, et qu'elles contiennent des ingrédients ou sont utilisées dans des conditions telles (notamment de pH) qui peuvent entraîner des réactions de sensibilisation de la peau, ainsi qu'une dégradation à la longue, des fibres traitées.

Des progrès importants ont été réalisés dans ces divers domaines de traitements des fibres kératiniques. Ainsi, les compositions contiennent de nombreux additifs qui, par exemple, visent à régler la rhéologie de la composition afin qu'elle puisse être appliquée facilement et rapidement, sans couler hors des zones à traiter pendant le temps de pose. Il existe aussi d'autres additifs qui ont pour objectif de limiter ou corriger des effets de dégradation de la fibres kératiniques occasionnés par les procédés.

Cependant, dans certains cas, les additifs présents ne sont pas sans effet sur les résultats mêmes du traitement.

C'est pourquoi on cherche toujours à améliorer la puissance, la sélectivité, la ténacité des colorations obtenues, l'homogénéité des décolorations, la tenue des formes données aux fibres.

Ainsi, la présente invention a pour objet de proposer des compositions qui résolvent les problèmes ci-dessus.

Un premier objet de l'invention est donc constitué par une composition destinée au traitement de matières kératiniques humaines, comprenant :
- au moins un composé choisi parmi l'acide éthylènediamine-N,N'-disuccinique, un sel, ou leur mélange,
- au moins un polymère épaississant choisi parmi les polymères à squelette aminoplaste-éther, les polymères cationiques associatifs, les homopolymères réticulés d'acide 2-acrylamido-2-méthylpropane sulfonique, ou les copolymères associatifs comprenant au moins un monomère à groupement sulfonique.

Un autre objet de la présente invention consiste à appliquer la composition selon l'invention sur des matières kératiniques, plus particulièrement humaines, et notamment sur des fibres.

Elle a de même pour objet des procédés de déformation permanente des fibres, de décoloration, de coloration, mettant en oeuvre la composition selon l'invention.

Mais d'autres caractéristiques et avantages de la présente invention apparaîtront plus clairement à la lecture de la description et des exemples qui vont suivre.

Dans ce qui va suivre et à moins que le contraire ne soit précisé, les bornes délimitant un domaine de valeurs sont comprises dans ce domaine.

Par ailleurs, par matières kératiniques humaines, on désigne la peau et les fibres kératiniques, et encore plus particulièrement des cheveux.

Dans le cas de compositions prêtes à l'emploi, dans le cas où celles-ci peuvent être stockées telles quelles, les teneurs détaillées pour les compositions selon l'invention restent valables, à l'exception du fait qu'elles sont exprimées par rapport à la composition prête à l'emploi.

Dans le cas de compositions prêtes à l'emploi obtenues par mélange extemporané de plusieurs compositions, alors ces teneurs sont à modifier en fonction du taux de dilution pour obtenir ladite composition prête à l'emploi.

Ainsi que cela a été indiqué auparavant, la composition selon l'invention comprend au moins un composé choisi parmi l'acide éthylènediamine-N,N'-disuccinique, un sel, ou leur mélange.

Les sels des composés sont des sels de métaux alcalins (comme par exemple le sodium, le potassium), de métaux alcalino-terreux (notamment le calcium, le magnésium), d'ammonium substitués ou non par un à trois radicaux alkyle, identiques ou différents, comprenant 1 à 6 atomes de carbone, de préférence de 1 à 4 atomes de carbone, éventuellement porteurs d'au moins un radical hydroxyle.

De préférence, le composé entrant dans la composition selon l'invention se présente sous la forme d'un sel de sodium.

Conformément à un mode de réalisation particulier de l'invention, la teneur en acide éthylènediamine-N,N'-disuccinique, sous forme acide ou sel, ou mélange, représente entre 0,01 et 20 % en poids par rapport au poids de la composition, plus particulièrement entre 0,1 et 10 % en poids par rapport au poids de la composition, de préférence entre 0,2 et 5 % en poids par rapport au poids de la composition.

La composition selon l'invention comprend par ailleurs au moins un polymère épaississant choisi parmi les polymères à squelette aminoplaste-éther, les polymères cationiques associatifs, les homopolymères réticulés d'acide 2-acrylamido-2-méthylpropane sulfonique, ou les copolymères associatifs comprenant au moins un monomère à groupement sulfonique.

Il est à noter qu'au sens de la présente invention, un polymère est qualifié d'associatif lorsqu'il possède au moins une partie hydrophile et au moins une partie hydrophobe et que les chaînes de polymères ont la capacité de s'associer entre elles (liaisons interpolymères) ainsi qu'avec d'autres composants de la composition, comme par exemple les tensioactifs.

Plus particulièrement, on désigne par "partie hydrophobe" une chaîne comprenant de 6 à 50 atomes de carbone, avantageusement de 6 à 22 atomes de carbone, de préférence de 8 à 18 atomes de carbone. Elle peut être linéaire ou ramifiée, et éventuellement porter une ou plusieurs insaturations éthyléniques (doubles liaisons carbone-carbone).

De manière plus avantageuse, ladite partie hydrophobe correspond à un radical choisi parmi les radicaux alkyle, arylalkyle, alkylaryle. De manière plus spécifique, la partie aryle de ces radicaux désigne des groupements phényle, benzyle, naphtyle ou anthryle.

En ce qui concerne les polymères épaississants du type des aminoplaste-éther, on désigne tout produit issu de la condensation d'un aldéhyde avec une amine ou un amide, ainsi que toute unité structurale formée d'un résidu aminoplaste et d'un résidu hydrocarboné divalent lié au résidu aminoplaste par une liaison éther.

Les polymères à squelette aminoplaste-éther présents dans la composition selon l'invention, sont choisis de préférence parmi ceux contenant au moins un motif de structure (I) suivante : 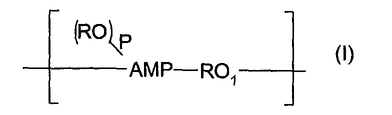 dans laquelle :
- AMP est un résidu aminoplaste avec des unités alkylènes (ou alkyle divalent),
- R désigne un atome d'hydrogène, un radical alkyle C₁-C₄ ou un radical acyle C₁-C₄,
- RO₁ est un résidu alkylène-oxy divalent,
- p désigne un nombre entier positif,
- le ou les groupements OR étant liés aux unités alkylènes du résidu AMP.

De préférence, les polymères à squelette aminoplaste-éther sont choisis parmi ceux contenant au moins un motif de structure (II) suivante : 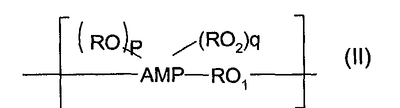 dans laquelle :
- AMP, R, RO₁ et p ont la même signification que précédemment,
- RO₂ est un groupe différent de RO lié à AMP au moyen d'un hétéroatome et comprenant au moins deux atomes de carbone, et,
- q est un nombre entier positif.

Plus préférentiellement encore, les polymères correspondent aux formules (III) et (III)bis suivantes : 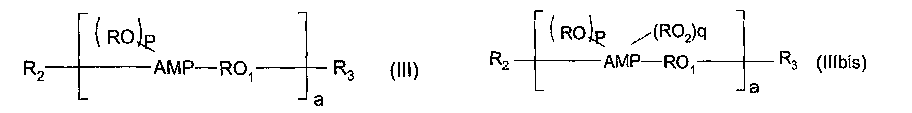 dans lesquelles :
- AMP, R, RO₁, RO₂, p et q ont la même signification que précédemment,
- R₂ ou R₃, identiques ou différents représentent un groupe terminal pouvant désigner un atome d'hydrogène, un groupement RO₁H, un groupement RO₂H, un groupement AMP(OR)p ou tout groupement monofonctionnel tel que alkyle, cycloalkyle, aryle, aralkyle, alkylaryle, alkyloxyalkyle, aryloxyalkyle, cycloalkoxyalkyle,
- a étant un nombre supérieur à 1 et de préférence supérieur à 2.

Les résidus aminoplastes porteurs de leurs groupements OR intégrés dans les polymères peuvent être choisis de manière non limitative parmi les structures (IV) à (XV) suivantes : 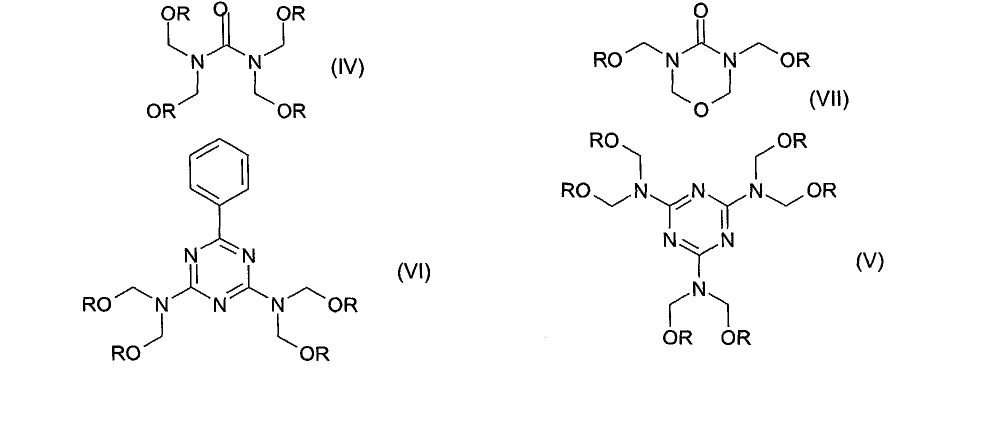 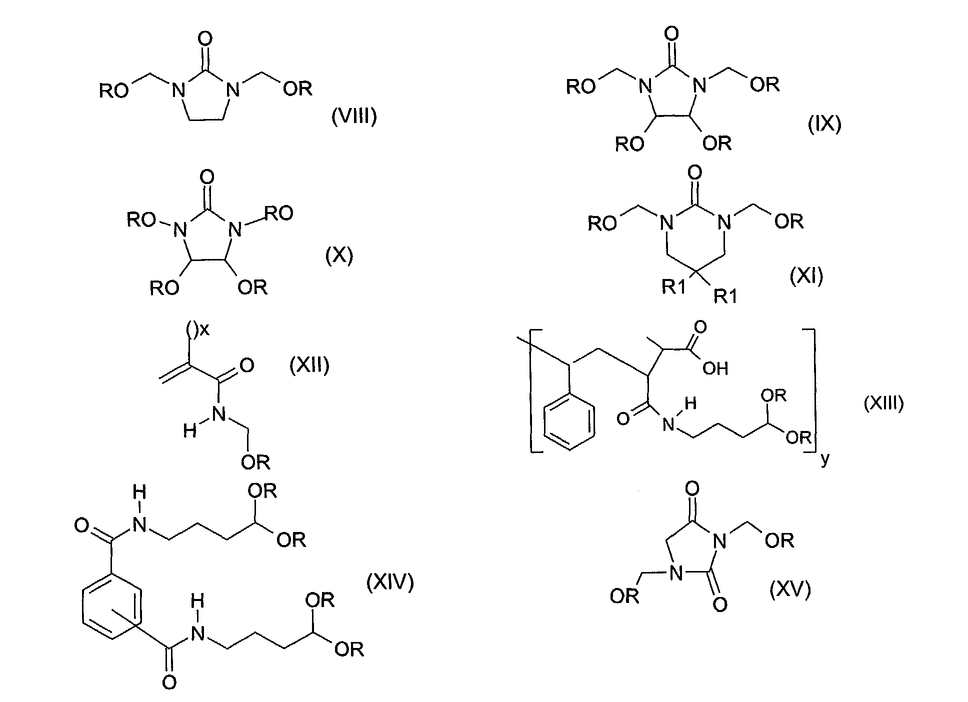 dans lesquelles :
- R a la même signification que précédemment,
- R₁ désigne alkyle C₁-C₄,
- y est un nombre au moins égal à 2,
- x désigne 0 ou 1.
   De préférence, le ou les résidus aminoplastes porteurs de leurs groupements OR sont choisis parmi ceux de structure (XVI) suivante : 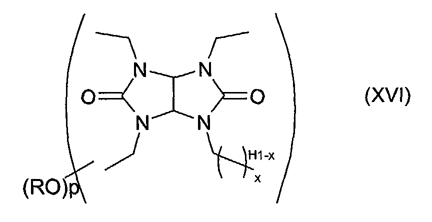
dans laquelle R, p , et x ont les mêmes significations que précédemment.

Les résidus alkylène-oxy divalents sont de préférence ceux correspondants aux diols de formule générale (XVII) suivante :

HO-(ZO)_{y}-(Z₁(Z₂O)_{w})ₜ-(Z'O)y'-Z₃OH (XVII),

- y et y' étant des nombres allant de 0 à 1000,
- t et w étant des nombres allant de 0 à 10,
- Z, Z', Z₂ et Z₃ sont des radicaux alkylène en C₂C₄ et de préférence des radicaux -CH₂-CH(Z₄)- et -CH₂-CH(Z₄)-CH₂-,
- Z₁ étant un radical linéaire ou cyclique, ramifié ou non, aromatique ou non, comportant ou non un ou plusieurs hétéroatomes et possédant de 1 à 40 atomes de carbone,
- Z₄ désignant un atome d'hydrogène ou un radical alkyle en C₁-C₄ ou un radical acyle en C₁-C₃ étant entendu qu'au moins un des radicaux Z₄ des radicaux Z, Z', Z₂ et Z₃ est différent d'un radical acyle.

De préférence Z₄ désigne un atome d'hydrogène ou un radical méthyle.

Encore plus préférentiellement t=0 et Z, Z' et Z₃ désignent -CH₂CH₂-, et l'un au moins de y ou y' est différent de 0. Les composés de formule (XVII) sont alors des polyéthylèneglycols.

Les polymères aminoplaste éther de formule (I) selon l'invention sont en particulier décrits dans le brevet US 5 914 373 auquel on pourra se référer pour plus de détails.

Comme polymères à squelette aminoplaste-éther de formule (I), on peut en particulier citer les produits Pure-Thix® L [PEG-180/Octoxynol-40/TMMG Copolymer (Nom INCI)], Pure-Thix M® [PEG-180/Laureth-50/TMMG Copolymer (Nom INCI)], Pure-Thix® HH [Polyether-1 (Nom INCI)] ; Pure Thix TX-1442® [copolymère PEG-18 / dodoxynol-5 / PEG-25 tristyrylphénol / tétraméthoxy méthyl glycolurile] proposés par la société Süd-Chemie.

Les polymères épaississants entrant dans la composition selon l'invention peuvent aussi être choisis parmi les polyuréthanes associatifs cationiques.

A titre d'exemples de polymères convenables, on peut citer par exemple ceux décrits dans le brevet FR 0009609.

Ces polymères correspondent plus particulièrement à la formule générale (XVIII) suivante :

R-X-(P)ₙ-[L-(Y)ₘ]ᵣL'-(P')ₚ-X'-R' (XVIII)

dans laquelle :
- R et R', identiques ou différents, représentent un groupement hydrophobe ou un atome d'hydrogène ;
- X et X', identiques ou différents, représentent un groupement comportant une fonction amine portant ou non un groupement hydrophobe, ou encore le groupement L" ;
- L, L' et L", identiques ou différents, représentent un groupement dérivé d'un diisocyanate ;
- P et P', identiques ou différents, représentent un groupement comportant une fonction amine portant ou non un groupement hydrophobe ;
- Y représente un groupement hydrophile ;
- r est un nombre entier compris entre 1 et 100, de préférence entre 1 et 50 et en particulier entre 1 et 25 ;
- n, m, et p valent chacun indépendamment des autres entre 0 et 1000 ;
- la molécule contenant au moins une fonction amine protonée ou quaternisée et au moins un groupement hydrophobe.

Dans un mode de réalisation très avantageux, les seuls groupements hydrophobes de ces polyuréthanes sont les groupes R et R' situés aux extrémités de chaîne.

Selon un premier mode de réalisation préféré, le polyuréthane associatif cationique correspondant à la formule (XVIII) décrite ci-dessus et dans laquelle :
- R et R' représentent tous les deux indépendamment un groupement hydrophobe,
- X, X' représentent chacun un groupe L",
- n et p valent entre 1 et 1000 et
- L, L', L", P, P', Y et m ont la signification indiquée que dans la formule (XVIII).

Selon un autre mode de réalisation préféré, le polyuréthane associatif cationique correspond à la formule (XVIII) ci-dessus dans laquelle :
- R et R' représentent tous les deux indépendamment un groupement hydrophobe,
- X, X' représentent chacun un groupe L",
- n et p valent 0,
- L, L', L", Y et m ont la signification que dans la formule (XVIII).

Il est à noter que lorsque n et p valent 0, cela signifie que ces polymères ne comportent pas de motifs dérivés d'un monomère à fonction amine, incorporé dans le polymère lors de la polycondensation.

Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Conformément à un autre mode de réalisation préféré, le polyuréthane associatif amphiphile cationique correspond à la formule (XVIII) ci-dessus dans laquelle :
- R et R' représentent tous les deux indépendamment un groupement hydrophobe,
- X et X' représentent tous les deux indépendamment un groupement comportant une amine quaternaire,
- n et p valent zéro, et
- L, L', Y et m ont la signification indiquée dans la formule (XVIII).

La masse moléculaire moyenne en nombre des polyuréthanes associatif amphiphile cationiques est habituellement comprise entre 400 et 500000, en particulier entre 1000 et 400000 et idéalement entre 1000 et 300000 g/mol.

Lorsque X et/ou X' désignent un groupement comportant une amine tertiaire ou quaternaire, X et/ou X' peuvent représenter l'une des formules suivantes : 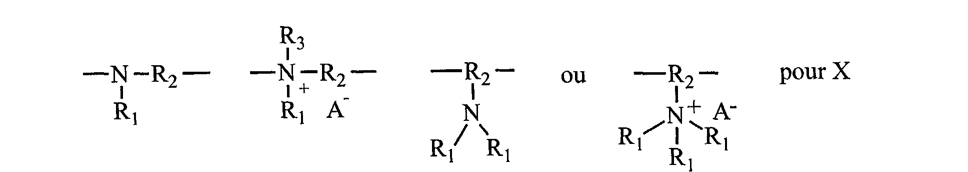 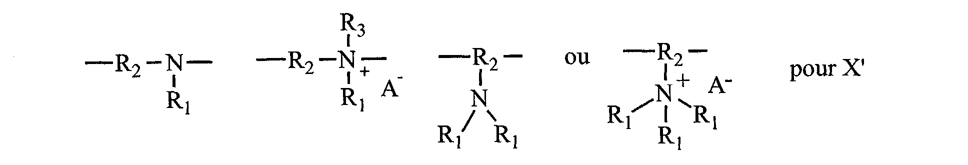 dans lesquelles :
- R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant chacun être remplacé par un hétéroatome choisi parmi N, S, O, P ;
- R₁ et R₃, identiques ou différents, désignent un radical alkyle ou alcényle en C₁-C₃₀, linéaire ou ramifié, un radical aryle, l'un au moins des atomes de carbone pouvant chacun être remplacé par un hétéroatome choisi parmi N, S, O, P ;
- A⁻ est un contre-ion cosmétiquement acceptable.

Les groupements L, L'et L" représentent un groupe de formule : 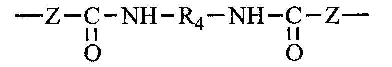 dans laquelle :
- Z représente -O-, -S- ou -NH- ; et
- R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant chacun être remplacé par un hétéroatome choisi parmi N, S, O et P.

Les groupements P et P', comprenant une fonction amine peuvent représenter au moins l'une des formules suivantes : 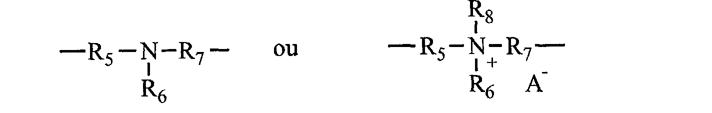 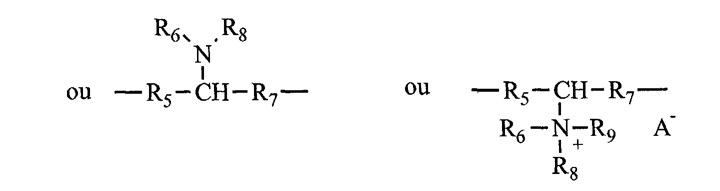 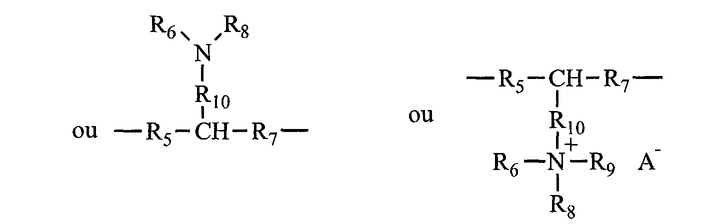 dans lesquelles :
- R₅ et R₇ ont les mêmes significations que R₂ défini précédemment ;
- R₆, R₈ et R₉ ont les mêmes significations que R₁ et R₃ définis précédemment ;
- R₁₀ représente un groupe alkylène, linéaire ou ramifié, éventuellement insaturé et pouvant contenir un ou plusieurs hétéroatomes choisis parmi N, O, S et P, et
- A⁻ est un contre-ion cosmétiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) et/ou poly(oxyde de propylène).

Les polymères épaississants cationiques entrant dans la composition selon l'invention peuvent aussi être choisis parmi les polyvinyllactames cationiques associatifs.

Parmi les polymères convenables, on peut citer entre autres ceux décrits dans le brevet FR 0101106.

Ces polymères sont plus particulièrement des polymères cationiques comprenant
a) au moins un monomère de type vinyllactame ou alkylvinyllactame;
b) au moins un monomère de structures (XIX) ou (XX) suivantes : 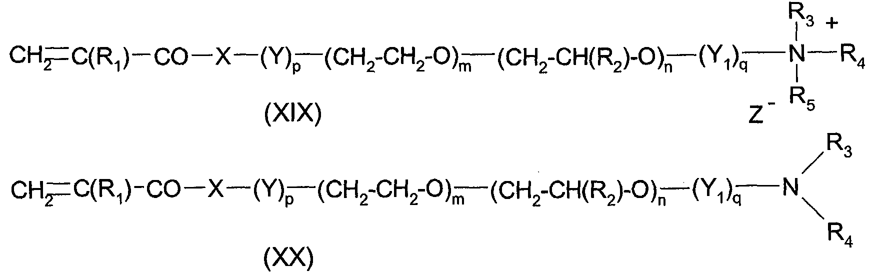
dans lesquelles :
- X désigne un atome d'oxygène ou un radical NR'₆,
- R₁ et R₆ désignent, indépendamment l'un de l'autre, un atome d'hydrogène ou un radical alkyle linéaire ou ramifié en C₁-C₅,
- R₂ désigne un radical alkyle linéaire ou ramifié en C₁-C₄,
- R₃, R₄ et R₅ désignent, indépendamment l'un de l'autre, un atome d'hydrogène, un radical alkyle linéaire ou ramifié en C₁-C₃₀ ou un radical de formule (XXI) : 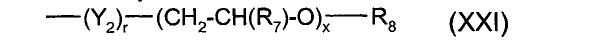
- Y, Y₁ et Y₂ désignent, indépendamment l'un de l'autre, un radical alkylène linéaire ou ramifié en C₂-C₁₆,
- R₇ désigne un atome d'hydrogène, ou un radical alkyle linéaire ou ramifié en C₁-C₄ ou un radical hydroxyalkyle linéaire ou ramifié en C₁-C₄,
- R₈ désigne un atome d'hydrogène ou un radical alkyle linéaire ou ramifié en C₁-C_{30,}
- p, q et r désignent, indépendamment l'un de l'autre, soit la valeur zéro, soit la valeur 1,
- m et n désignent, indépendamment l'un de l'autre, un nombre entier allant de 0 à 100,
- x désigne un nombre entier allant de 1 à 100,
- Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0.

Les polymères poly(vinyllactame) présents dans la composition selon l'invention peuvent être réticulés ou non réticulés et peuvent aussi être des polymères blocs.

De préférence le contre ion Z- des monomères de formule (XIX) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (XIX) pour laquelle, encore plus préférentiellement, m et n sont égaux à zéro.

Le monomère vinyllactame ou alkylvinyllactame est de préférence un composé de structure (XXII) : 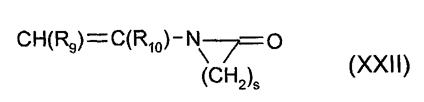 dans laquelle :
- s désigne un nombre entier allant de 3 à 6,
- R₉ désigne un atome d'hydrogène ou un radical alkyle linéaire ou ramifié en C₁-C₅,
- R₁₀ désigne un atome d'hydrogène ou un radical alkyle linéaire ou ramifié en C₁-C₅,
- sous réserve que l'un au moins des radicaux R₉ et R₁₀ désigne un atome d'hydrogène.

De manière encore plus avantageuse, le monomère (XXII) est la vinylpyrrolidone.

Lesdits polymères poly(vinyllactame) peuvent également contenir un ou plusieurs monomères supplémentaires, de préférence cationiques ou non ioniques.

A titre de composés plus particulièrement préférés, on peut citer les terpolymères suivants comprenant au moins :
a) un monomère de formule (XXII),
b) un monomère de formule (XIX) dans laquelle p=1, q=0, R₃ et R₄ désignent, indépendamment l'un de l'autre, un atome d'hydrogène ou un radical alkyle en C₁-C₅, et R₅ désigne un radical alkyle en C₉-C₂₄ et
c) un monomère de formule (XX) dans laquelle R₃ et R₄ désignent, indépendamment l'un de l'autre, un atome d'hydrogène ou un radical alkyle en C₁-C₅.

Encore plus préférentiellement, on met en oeuvre des terpolymères comprenant, en poids, 40 à 95% de monomère (a), 0,1 à 55% de monomère (c) et 0,25 à 50% de monomère (b).

De tels polymères sont notamment décrits dans la demande de brevet WO 00/68282 auquel on pourra se référer pour plus de détails.

Comme polymères poly(vinyllactame), on peut utiliser notamment les terpolymères vinylpyrrolidone / diméthylaminopropylméthacrylamide / tosylate de dodécyl diméthyl méthacrylamidopropylammonium, les terpolymères vinylpyrrolidone / diméthyl amino propylméthacrylamide / tosylate de cocoyldiméthylméthacrylamido propyl ammonium, les terpolymères vinylpyrrolidone / diméthylaminopropyl méthacrylamide / tosylate ou chlorure de lauryidiméthylméthacrylamidopropylammonium.

Le terpolymère vinylpyrrolidone / diméthylaminopropyl méthacrylamide /chlorure de lauryl diméthylméthacrylamido propylammonium est proposé dans l'eau à 20% par la Société ISP sous la dénomination Styleze W20®.

Les polymères épaississants cationiques entrant dans la composition selon l'invention peuvent aussi être choisis parmi les polymères amphiphiles dérivés de celluloses associatifs comme notamment :
- les celluloses cationiques quaternisées modifiées par des groupements comportant au moins une partie hydrophobe telle que définie auparavant,
- les hydroxyéthylcelluloses cationiques quaternisées modifiées par des groupements comportant au moins une partie hydrophobe définie auparavant.

On peut indiquer comme exemples d'alkylhydroxyéthylcelluloses quaternisées dont la partie hydrophobe est en C₈-C₃₀, les produits Quatrisoft LM 200®, Quatrisoft LM-X 529-18-A®, Quatrisoft LM-X 529-18B® (alkyle en C₁₂) et Quatrisoft LM-X 529-8® (alkyle en C₁₈) commercialisés par la société Amerchol et les produits Crodacel QM®, Crodacel QL® (alkyle en C₁₂) et Crodacel QS® (alkyle en C₁₈) commercialisés par la société Croda.

Les polymères épaississants entrant dans la composition selon l'invention peuvent aussi être choisis parmi les homopolymères réticulés d'acide 2-acrylamido-2-méthylpropane sulfonique, ou les copolymères associatifs comprenant au moins un monomère du type de l'acide vinylsulfonique, l'acide styrènesulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique ; l'acide 2-acrylamido-2-méthylpropane sulfonique étant préféré.

En ce qui concerne les homopolymères réticulés d'acide 2-acrylamido-2-méthyl-propane sulfonique et leurs copolymères réticulés d'acrylamide qui peuvent être partiellement ou totalement neutralisés, on peut citer les polymères comprenant de 90 à 99,9% en poids, par rapport au poids total du polymère, de motifs de formule (XXIII) suivante : 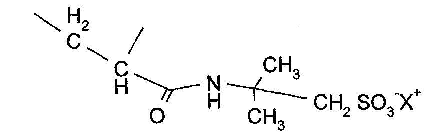 dans laquelle X⁺ désigne un cation ou un mélange de cations, ou un proton.

Plus particulièrement les cations sont choisis parmi les métaux alcalins (comme le sodium, le potassium), les ions ammonium substitués ou non par un à trois radicaux alkyle, identiques ou différents, comprenant 1 à 6 atomes de carbone, éventuellement porteur d'au moins un radical hydroxyle, les cations dérivant de la N-méthyl-glucamine, d'acides aminés basiques comme l'arginine et la lysine. De préférence, le cation est un ion ammonium ou sodium.

Par ailleurs, le polymère comprend de 0,01 à 10% en poids, par rapport au poids total du polymère, de motifs réticulants provenant d'au moins un monomère ayant au moins deux insaturation éthyléniques (double liaison carbone-carbone).

Les monomères de réticulation ayant au moins deux insaturations éthyléniques sont choisis par exemple parmi l'éther diallylique, le triallylcyanurate, le diallylmaléate, le (méth)acrylate d'allyle, le dipropylèneglycol-diallyléther, les polyglycol-diallyléthers, le triéthylèneglycol-divinyléther, l'hydroquinone-diallyl-éther, le tétrallyl-oxéthanoyle, le di(méth)acrylate de tétra- ou di-éthylèneglycol, la triallylamine, la tétraallyléthylènediamine le triméthylolpropane-diallyléther, le triméthylolpropane triacrylate, le méthylène-bis(méth)acrylamide ou le divinylbenzène, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Pour plus de détail au sujet de ces polymères, on pourra se reporter au document EP 815828.

Parmi les copolymères réticulés d'acide 2-acrylamido-2-méthyl-propane sulfonique et d'acrylamide partiellement ou totalement neutralisés, on peut citer en particulier le produit décrit dans l'exemple 1 du document EP 503 853 et l'on pourra se reporter à ce document pour ce qui a trait à ces polymères.

Les polymères épaississants entrant comme ingrédient dans la composition selon l'invention peuvent aussi être choisis parmi les polymères associatifs comportant au moins un monomère à insaturation éthylénique à groupement sulfonique, sous forme libre ou partiellement ou totalement neutralisée et comprenant au moins une partie hydrophobe.

De façon préférentielle, lesdits polymères sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Ces polymères associatifs peuvent être ou non réticulés, et de préférence sont des polymères réticulés. Dans ce cas, les agents réticulants peuvent notamment être choisis parmi les agents listés précédemment dans le cadre de la description des homopolymères réticulés d'acide 2-acrylamido-2-méthyl-propane sulfonique. On utilise plus particulièrement le méthylène-bis-acrylamide, le méthacrylate d'allyle ou le triméthylol propane triacrylate. Le taux de réticulation varie en général de 0,01 à 10% en mole, par rapport au polymère.

Les monomères à insaturation éthylénique à groupement sulfonique sont choisis notamment parmi l'acide vinylsulfonique, l'acide styrènesulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique ainsi que leurs formes partiellement ou totalement neutralisées.

Plus particulièrement, on peut utiliser les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

On utilise de préférence l'acide 2-acrylamido-2-méthylpropane-sulfonique (AMPS) ainsi que ses formes partiellement ou totalement neutralisées.

Les polymères amphiphiles présents dans la composition selon l'invention peuvent aussi être choisis parmi les polymères amphiphiles statistiques d'AMPS modifiés par réaction avec une n-monoalkylamine ou une di-n-alkylamine en C₆-C₂₂, et tels que ceux décrits dans la demande WO00/31154.

Les monomères hydrophobes qui constituent la partie hydrophobe du polymère sont choisis de préférence parmi les acrylates ou les acrylamides de formule (XXIV) suivante : 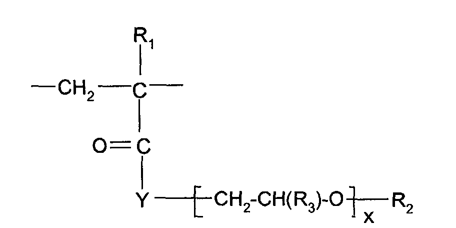 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe tel que défini auparavant ; x désigne un nombre de moles d'oxyde d'alkylène et varie de 0 à 100.

Le radical R₂ est choisi avantageusement parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈ (par exemple le groupement de formule -(CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulière de l'invention, le monomère de formule (XXIV) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Les copolymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans les documents EP750899, US 5089578, les publications de Yotaro Morishima suivantes : «Self-assembling amphiphilic polyelectrolytes and their nanostructures - Chinese Journal of Polymer Science Vol. 18, N°40, (2000), 323-336.» ; «Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704» ; «Solution properties of miscelle networks formed by non-ionic moieties covalently bound to an polyelectrolyte: salt effects on rheological behaviour- Langmuir, 2000, Vol.16, N°12, 5324-5332» ; «Stimuli responsive amphiphilic copolymers of sodium 2-(acrylamido)-2-methylpropanesulfonate and associative macromonomers - Polym. Preprint, Div. Polym. Chem. 1999,40(2),220-221».

La répartition des monomères dans le copolymère peut être statistique ou bloc.

Parmi ces polymères de ce type, on peut citer plus spécialement :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, tels que ceux décrits dans le brevet US- 5089578.
- les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs AMPS de formule (XXV) suivante : 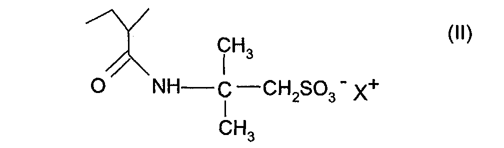 dans laquelle X⁺ a la même définition que précédemment,
et de motifs de formule (XXVI) suivante : 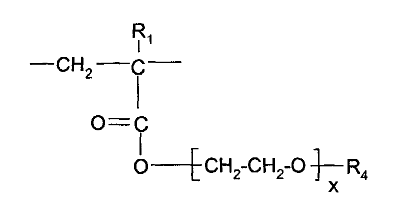 dans laquelle x désigne un nombre entier variant de 3 à 100, de préférence de 5 à 80 et plus préférentiellement de 7 à 25 ; R₁ a la même signification que celle indiquée ci-dessus dans la formule (XXIII) et R₄ désigne un alkyle linéaire ou ramifié en C₆-C₂₂ et plus préférentiellement en C₁₀-C₂₂.

Les polymères particulièrement préférés sont ceux pour lesquels x = 25, R₁ désigne méthyle et R₄ représente n-dodécyle ; ils sont décrits dans les articles de Morishima mentionnés ci-dessus.

Les polymères pour lesquels X⁺ désigne sodium ou ammonium sont plus particulièrement préférés.

Les polymères de la gamme Genapol® de la société Hoechst/Clariant peuvent être employés dans la composition selon l'invention.

Selon un mode de réalisation particulier de l'invention, la teneur en polymère épaississant représente 0,01 à 20 % en poids par rapport au poids de la composition, de préférence entre 0,1 et 10 % en poids par rapport au poids de la composition, et de manière encore plus avantageuse, entre 0,5 et 5 % en poids par rapport au poids de la composition.

La composition selon l'invention peut en outre comprendre au moins un agent oxydant.

Habituellement, l'agent oxydant est choisi parmi le peroxyde d'hydrogène, les peroxydes de métaux alcalins ou alcalino-terreux, comme le sodium, le potassium, le magnésium ; le peroxyde d'urée, les bromates ou ferricyanures de métaux alcalins, les persels tels que les perborates et les persulfates de métaux alcalins ou alcalino-terreux, comme le sodium, le potassium, le magnésium, seuls ou en mélanges.

La teneur en agent oxydant est en général comprise entre 1 et 60 % en poids, de préférence entre 3 et 50 % en poids par rapport au poids de la composition.

La composition comprenant l'agent oxydant peut se trouver sous la forme d'une solution, d'une émulsion, ou encore d'une pâte.

Dans ce dernier cas, la pâte ne comprend pas d'eau ou en comprend une teneur inférieure ou égale à 1 % en poids par rapport au poids de la composition, de préférence inférieure ou égale à 0,5 % en poids par rapport au poids de la composition.

Au cas où la composition oxydante est destinée à être utilisée pour la décoloration ou le décapage de la fibre kératinique, elle comprend alors de préférence au moins un persel, au moins un agent alcalin et de 15 à 35 % d'au moins un liquide inerte organique.

Usuellement, l'agent alcalin est choisi parmi l'urée ; les sels d'ammonium, comme les chlorures, les sulfates, les phosphates, les nitrates ; les silicates, les phosphates, les carbonates de métaux alcalins (tels que le sodium, le potassium) ou alcalino-terreux (comme notamment le magnésium) seuls ou combinés.

Il est en général présent à hauteur de 0,01 à 40 % en poids, de préférence de 0,1 à 30 % en poids, par rapport au poids de la composition.

Quant au liquide inerte organique, ce dernier est un composé ou un mélange de composés liquides à température ambiante (entre 15 et 25°C) et à pression atmosphérique. Avantageusement, il est choisi parmi les polydécènes, les mono- et poly- esters d'acides carboxyliques, les mono- ou poly- esters de sucres d'acides en C₈-C₃₀, les éthers cycliques, les esters cycliques, les huiles silicones, les huiles minérales, les huiles végétales, ou leurs mélanges.

Notons que des compositions sous forme de pâte sont obtenues par tout moyen classique, par exemple par dispersion des composés pulvérulents dans le liquide inerte, dans lequel on a préalablement dispersé ou mélangé les autres composés liquides de la composition de décoloration, ou encore par extrusion.

La composition selon l'invention peut comprendre par ailleurs au moins un agent réducteur, que ce soit pour le décapage ou pour la déformation permanente des fibres.

Plus particulièrement, l'agent réducteur est choisi parmi les réductones ou les composés comprenant du soufre, et notamment des composés présentant au moins une fonction thiol, (di)sulfure, sulfite, sulfinique, sous forme de sel ou non.

Parmi les réductones, on peut citer entre autres l'acide (iso)ascorbique, l'acide érythorbique, sous forme acide, estérifiée ou encore salifiée. De manière particulièrement avantageuse conviennent l'acide ascorbique, l'acide isoascorbique, sous forme acide ou de l'un de ses sels comme le sel de sodium.

Parmi les thiols utilisables en tant que composé réducteur, on peut citer l'acide thioglycolique, le β-mercaptoéthanol, l'acide thiolactique, leurs sels de métal alcalin ou alcalino-terreux (comme le sodium, le potassium, le calcium) et leurs esters ; la cystéine, la cystéamine et leurs dérivés ; l'homocystéine et l'un de ses sels ; le mercaptoaldéhyde ; la pénicillamine ; la glutathione ; le thioglycolate de glycérol. Ces composés pouvant être utilisés seuls ou en mélanges.

En ce qui concerne les sulfites utilisables en tant que réducteurs, parmi lesquels figurent aussi les bisulfites, les hydrosulfites, conviennent les sels de métaux alcalins, alcalino-terreux ou d'ammonium, ainsi que leurs mélanges. Plus particulièrement, on peut citer le sulfite de sodium et l'hydrosulfite de sodium.

En ce qui concerne les sulfures, parmi lesquels figurent aussi les disulfures, on peut citer notamment la cystine.

Pour ce qui a trait aux composés sulfiniques, on peut citer les composés correspondants à la formule suivante : 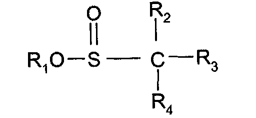 dans laquelle,
R₁ est choisi parmi, un atome d'hydrogène, un ion NH₃⁺, un ion de métal monovalent ou un équivalent ionique de métal bivalent des groupes Ia, IIa, IIb, IVa et VIIIb du système périodique des éléments,
R₂ est choisi parmi, un radical OH, un radical NR₅R₆ dans lequel R₅ et R₆, identiques ou différents, sont choisis parmi un atome d'hydrogène ou un radical alkyle en C₁-C₆,
R₃ est choisi parmi, un atome d'hydrogène, un radical alkyle ou alcényle ou cycloalkyle en C₁-C₆ ; aryle non substitué ou substitué par 1 à 3 substituants choisis, identiques ou différents, parmi les radicaux OH, alkyle en C₁-C₆, O-alkyle en C₁-C₆, halogène ou CF₃, R₄ est choisi parmi, un radical COOR₁, SO₃R₁, COR₅, CONR₅R₆ ou COOR₅ ou dans lesquels R₁, R₅, et R₆ ont les significations précédentes,
et R₄ désigne également un atome d'hydrogène, lorsque R₃ désigne un radical aryle, et en particulier, un radical aryle substitué comme décrit précédemment,
et les sels desdits composés cosmétiquement acceptables.

De préférence, les sels des composés sulfiniques peuvent être choisis parmi les sulfinates alcalins (Na, K), alcalinoterreux (Ca, Mg) ou de zinc.

Les dérivés d'acides sulfiniques de formule ci-dessus selon l'invention sont des composés connus, décrits et préparés dans la demande de brevet WO 99/18067 et WO02/30369.

De manière avantageuse, on peut mettre en oeuvre des dérivés d'acide sulfinique de formule précitée pour laquelle R₁ est choisi parmi un ion NH₃⁺, un ion de métal alcalin, un équivalent ionique de métal alcalino-terreux ou de zinc, R₂ est un radical OH ou un radical NH₂, R₃ est choisi parmi, un atome d'hydrogène, un radical alkyle non substitué ou substitué par un ou deux radicaux OH ou un ou deux radicaux alkyle en C₁-C₆ ou alcoxy en C₁-C₆, R₄ est un radical COOR₁ ou COOR₅, dans lesquels R₁ et R₅ ont les mêmes significations que celles indiquée plus haut.

Conformément à un mode de réalisation plus particulier, le dérivé d'acide sulfinique est tel que R₁ représente le sodium, R₂ représente OH, R₃ représente un atome d'hydrogène et R₄ représente COONa. Sont aussi susceptibles de convenir, des mélanges comprenant 40 à 60% dudit composé, 10 à 20% de NaSO₃-CHOH-COONa et de 30 à 40% de Na₂SO₃ (par exemple le produit Brüggolite® FF6 de la société Bruggemann : 50% du composé NaSO₂-CHOH-COONa ; 15% du composé NaSO₃-CHOH-COONa et 35% de Na₂SO₃).

On pourra aussi mettre en oeuvre à titre d'agent réducteur, les dérivés d'acide sulfinique notamment décrits dans WO03/026597 et WO03/041668.

De préférence, l'agent réducteur est choisi parmi l'acide ascorbique, l'hydroxyméthane sulfinate de sodium, les thiols, le sulfite de sodium, seuls ou en mélanges.

La teneur en agent réducteur dans la composition est avantageusement comprise entre 0,01 et 10 % en poids par rapport au poids de la composition, de préférence entre 0,1 et 5 % en poids par rapport au poids de la composition.

Notons que la composition peut se trouver sous la forme d'une lotion épaissie, d'une crème, d'un gel, ou de toute autre forme appropriée pour l'application ultérieure de cette composition.

Ainsi, lorsque la composition est destinée à une opération de défrisage ou de décrêpage de fibres, elle se trouve de préférence sous forme d'une crème épaissie de façon à maintenir les fibres à traiter aussi raides que possible pendant le traitement. On réalise ces crèmes sous forme d'émulsions "lourdes", obtenues par exemple en émulsionnant une phase aqueuse, comprenant notamment l'agent réducteur, et une phase huileuse (huile végétale, huile de paraffine, esters d'acides gras, cire, par exemple).

On peut également utiliser des liquides ou des gels contenant des agents épaississants tels que des polymères ou des copolymères carboxyvinyliques qui "collent" les fibres et les maintiennent dans la position lisse pendant le temps de pose.

Il est à noter que la composition peut de même comprendre au moins un colorant direct, au moins une base d'oxydation éventuellement associée à au moins un coupleur, ou leurs mélanges.

En ce qui concerne les colorants directs, on peut utiliser des espèces anioniques, cationiques ou non ioniques.

Avantageusement, ils peuvent être choisis parmi les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane, les colorants naturels, seuls ou en mélanges.

S'ils sont présents, la teneur en colorant(s) direct(s) dans la composition varie en général de 0,001 à 20% en poids par rapport à la composition, et de préférence de 0,01 à 10% du poids total de la composition.

En ce qui concerne les bases d'oxydation, elles peuvent être choisies notamment parmi les o-phénylènediamines, les p-phénylènediamines, les bis-phénylènediamines, les o-aminophénols, les p-aminophénols, les bis-para-aminophénols, les bases hétérocycliques, leurs sels d'addition avec un acide, ainsi que leurs mélanges.

En général, lorsqu'elles sont présentes dans la composition, la teneur en base(s) d'oxydation représente de 0,0005 à 12% en poids par rapport au poids de la composition, avantageusement de 0,005 à 8% en poids par rapport au poids de la composition.

Quant aux coupleurs éventuellement associées aux bases d'oxydation précitées, on peut mettre en oeuvre un ou plusieurs composés choisis parmi les m-aminophénols, les m-phénylènediamines, les m-diphénols, les naphtols, les coupleurs hétérocycliques, leurs sels d'addition avec un acide, ainsi que leurs mélanges.

Lorsqu'ils sont présents, ces coupleurs représentent plus spécialement de 0,0001 à 10% en poids par rapport au poids de la composition, et de préférence de 0,005 à 5% en poids par rapport au poids de la composition.

D'une manière générale, les sels d'addition avec un acide des bases d'oxydation et coupleurs sont notamment choisis parmi les chlorhydrates, les bromhydrates, les sulfates, les citrates, les succinates, les tartrates, les lactates, les tosylates, les benzènesulfonates, les phosphates et les acétates.

En outre, la composition selon l'invention peut de plus comprendre au moins un tensioactif, de préférence non ionique, anionique, amphotère ou zwittérionique.

Parmi les tensioactifs non ioniques, on peut citer les alcools, les alpha-diols, les alkylphénols polyéthoxylés et/ou polypropoxylés, ayant une chaîne hydrocarbonée comprenant par exemple de 8 à 30 atomes de carbone, de préférence de 8 à 18 atomes de carbone, le nombre de groupements oxyde d'éthylène et/ou oxyde de propylène pouvant aller notamment de 2 à 50.

On peut également citer les copolymères d'oxyde d'éthylène et de propylène, les condensats d'oxyde d'éthylène et/ou de propylène sur des alcools gras ; les amides gras polyéthoxylés ayant de préférence de 2 à 30 moles d'oxyde d'éthylène ; les esters d'acides gras de sorbitan oxyéthylénés ayant de 2 à 30 moles d'oxyde d'éthylène ; les esters d'acides gras de sucrose, les esters d'acides gras et de polyéthylèneglycol, les alkylpolyglycosides, les dérivés de N-alkyl glucamine, etc.

Parmi les tensioactifs anioniques, on peut citer entre autres les sels (en particulier les sels alcalins, notamment de sodium, de magnésium, les sels d'ammonium, les sels d'amines, les sels d'aminoalcools) d'alkylsulfates, alkyléthersulfates, alkylamidoéthersulfates, alkylarylpolyéthersulfates, monoglycérides sulfates ; alkylsulfonates, alkylphosphates, alkylamidesulfonates, alkylarylsulfonates, α-oléfine-sulfonates, paraffine-sulfonates ; alkyl(C₆-C₂₄) sulfosuccinates, alkyl(C₆-C₂₄) éthersulfosuccinates, alkyl(C₆-C₂₄) amidesulfosuccinates ; alkyl(C₆-C₂₄) suifoacétates ; acyl(C₆-C₂₄) sarcosinates et glutamates ; esters d'alkyl(C₆-C₂₄)polyglycosides carboxyliques ; alkylsulfosuccinamates ; acyliséthionates et N-acyltaurates ; le radical alkyle ou acyle de tous ces différents composés comportant de préférence de 12 à 20 atomes de carbone, et le radical aryle désignant de préférence un groupement phényle ou benzyle.

Conviennent aussi les sels d'acides gras tels que les sels des acides oléique, ricinoléique, palmitique, stéarique, les acides d'huile de coprah hydrogénée ou non ; les acides d'alkyl D-galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

En ce qui concerne les tensioactifs amphotères ou zwittérioniques conviennent notamment les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer amphocarboxyglycinates et amphocarboxypropionates, comme par exemple les disodium cocoamphodiacetate, disodium lauroamphodiacetate, disodium caprylamphodiacetate, disodium cocoamphodipropionate, disodium lauroamphodipropionate, etc. (produits de la gamme Miranol®, notamment Miranol® C2M concentré commercialisés la société Rhodia Chimie).

La teneur en tensioactif dans la composition représente habituellement de 0,01 à 40 % en poids et de préférence de 0,5 à 30 % en poids, du poids total de la composition.

La composition conforme à l'invention peut également comprendre divers adjuvants utilisés classiquement, tels que des polymères substantifs cationiques ou amphotères, des polymères anioniques, cationiques, non ioniques, amphotères, zwittérioniques, des agents épaississants minéraux, des agents antioxydants, des acides alpha-oxocarboxyliques (par exemple l'acide oxalique, l'acide glyoxalique, l'acide pyruvique ou l'acide alpha-cétoglutarique), des agents de pénétration, des parfums, des tampons, des acides aminés basiques comme l'arginine notamment, des agents dispersants, des agents peptisants, des agents de conditionnement tels que par exemple des cations, des silicones volatiles ou non volatiles, modifiées ou non modifiées, des agents filmogènes, des céramides, des vitamines ou provitamines, des agents conservateurs, des agents stabilisants, des agents opacifiants ou matifiants comme le dioxyde de titane, des charges minérales, comme les argiles, les silices notamment pyrogénées à caractère hydrophile ou hydrophobe, des polymères liants tels que la vinylpyrrolidone, des lubrifiants comme les stéarates, des agents de contrôle du dégagement d'oxygène tels que le carbonate ou l'oxyde de magnésium, les filtres solaires, etc.

Les adjuvants cités ci-dessus sont en général présents en quantité comprise pour chacun d'eux entre 0,01 et 20% en poids par rapport au poids de la composition.

Le milieu de la composition est un milieu cosmétiquement acceptable.

Selon une première possibilité, il est constitué par de l'eau ou par un mélange d'eau et d'un ou plusieurs solvants organiques usuels.

Plus particulièrement, les solvants organiques sont choisis parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants usuels décrits ci-dessus, s'ils sont présents, représentent habituellement de 1 à 40 % en poids, plus préférentiellement de 5 à 30 % en poids, par rapport au poids total de la composition.

Selon une deuxième possibilité, le milieu de la composition selon l'invention ne comprend pas d'eau ou comprend une teneur en eau inférieure ou égale à 1 % en poids par rapport au poids de la composition, de préférence inférieure ou égale à 0,5 % en poids par rapport au poids de la composition.

Dans ce cas, le milieu de la composition est un liquide organique inerte choisi parmi ceux mentionnés auparavant.

La teneur en ce type de composés est comprise entre 15 et 35 % en poids par rapport au poids de la composition.

Le pH de la composition selon l'invention peut être déterminé sans difficulté par l'homme du métier et dépend notamment de la nature des ingrédients présents mais aussi de l'application que l'on fait de ladite composition.

Il peut être ajusté à la valeur désirée au moyen d'agents acidifiants ou alcalinisants.

A titre indicatif, le pH de la composition selon l'invention est généralement compris entre 3 et 14 environ, et de préférence entre 5 et 12 environ.

Parmi les agents acidifiants, on peut citer, à titre d'exemples, les acides minéraux ou organiques comme l'acide chlorhydrique, l'acide orthophosphorique, l'acide sulfurique, l'acide acétique.

Parmi les agents alcalinisants on peut citer, à titre d'exemples, l'ammoniaque, les carbonates alcalins, les carbonates organiques, les alcanolamines telles que les mono-, di- et triéthanolamines ainsi que leurs dérivés, les hydroxydes de sodium ou de potassium et les composés de formule (A) suivante : 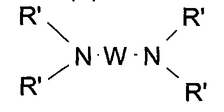 dans laquelle W est un reste propylène éventuellement substitué par un groupement hydroxyle ou un radical alkyle en C1-C6 ; les radicaux R', identiques ou différents, représentent un atome d'hydrogène, un radical alkyle en C₁-C₆ ou hydroxyalkyle en C₁-C₆.

La composition selon l'invention peut se trouver sous la forme de produits de lavage, comme des shampooings, mais aussi des après-shampooings, des produits de mise en forme (comme par exemple des laques, des mousse, etc.), des produits de coloration, de décoloration, de mise en forme des fibres kératiniques.

La présente invention a de plus pour objet des procédés mettant en oeuvre la composition selon l'invention.

Ainsi, le procédé consiste à mettre en contact les matières kératiniques, sèches ou humides, avec la composition selon l'invention qui vient d'être décrite.

Généralement, le temps de pose de la composition est compris entre quelques secondes et 30 minutes, de préférence entre 3 et 15 minutes.

La température à laquelle la composition est laissée agir est en général comprise entre la température ambiante (15 à 25°C) et 220°C, plus particulièrement entre la température ambiante et 80°C, de préférence entre 15 et 40 °C.

De préférence, les matières kératiniques sont rincées, avantageusement à l'eau, et si nécessaire, lavées avec un shampooing puis rincées à nouveau à l'eau

Selon un premier mode de réalisation particulier, l'invention a pour objet un procédé de déformation permanente des fibres kératiniques humaines consistant à mettre en contact dans une première étape, une composition réductrice avec lesdites fibres kératiniques, ces dernières étant mises en forme avant, pendant ou après l'application de la composition réductrice ; puis dans une deuxième étape, à appliquer une composition oxydante sur les fibres ainsi traitées ; le composé portant les quatre fonctions carboxyliques et le polymère épaississant se trouvant dans la composition réductrice et/ou dans la composition oxydante.

La première étape consiste donc à appliquer sur lesdites fibres, sèches ou humides, une composition réductrice. Cette composition peut éventuellement comprendre le composé portant les quatre fonctions carboxyliques et le polymère épaississant.

De préférence, selon cette première variante, le pH de la composition réductrice appliquée est d'au moins 7.

Si l'opération est destinée à friser les fibres, ces dernières sont mises sous tension au moyen de bigoudis, avant, pendant ou après l'application de la composition réductrice. En cas de défrisage, la composition est appliquée sur les fibres en les lissant.

Le temps de pose est en général compris entre quelques secondes et 30 minutes, de préférence entre 3 et 15 minutes.

La température à laquelle la composition est laissée agir est en général comprise entre la température ambiante (15 à 25°C) et 220°C, plus particulièrement entre la température ambiante et 80°C, de préférence entre 15 et 40 °C.

Il peut être approprié d'effectuer une étape intermédiaire, entre l'étape de réduction et celle de fixation (d'oxydation), d'appliquer de la chaleur.

Une fois le temps de pose écoulé et éventuellement cette étape intermédiaire réalisée, on applique sur les fibres une composition oxydante.

Comme indiqué auparavant, la composition oxydante peut éventuellement comprendre le composé portant les quatre fonctions carboxyliques et le polymère épaississant.

De manière classique, la composition comprend, à titre d'agent oxydant, du peroxyde d'hydrogène, un bromate alcalin, un persel ou un polythionate.

Le pH de cette composition oxydante est généralement compris entre 2 et 10.

De préférence, on utilise du peroxyde d'hydrogène avec un pH avantageusement compris entre 2 et 7.

La composition oxydante peut de plus être obtenue en mélangeant au moins deux compositions, l'une d'elles comprenant l'agent oxydant, l'autre comprenant au moins un agent alcalin.

La composition oxydante peut se trouver sous avantageusement sus la forme d'une émulsion, habituellement épaissie.

Le temps de pose de la composition oxydante est en général compris entre 3 et 30 minutes, de préférence entre 5 et 15 minutes.

Le plus souvent, les fibres imprégnées de la composition oxydante sont rincés, généralement à l'eau. On sépare, avant ou après le rinçage, les fibres kératiniques des moyens nécessaires à leur mise sous tension.

Les fibres sont ensuite éventuellement lavées au shampooing, rincées puis séchées ou laissées à sécher.

Ce qui a été indiqué plus haut quant à la nature des divers ingrédients entrant dans les compositions, ainsi que leurs quantités respectives, reste valable et ne sera pas repris dans cette partie de la description.

Conformément à une autre variante, le procédé de mise en forme de fibres kératiniques humaines consiste à appliquer sur les fibres kératiniques, sèches ou humides, une composition comprenant, en plus du ou des composés et polymères épaississants précités, au moins un agent acalinisant, tel que de préférence l'hydroxyde de sodium.

Ce type de composition est particulièrement approprié lorsque l'on souhaite faire une étape de défrisage des cheveux.

Habituellement, les fibres sont lissées pendant ou après l'application de la composition.

Le temps de pose est en général compris entre 3 et 30 minutes, de préférence entre 5 et 15 minutes.

La température à laquelle la composition est laissée agir est en général comprise entre la température ambiante (15 à 25°C) et 220°C, plus particulièrement entre la température ambiante et 80°C, de préférence entre 15 et 40 °C.

Les fibres imprégnées de la composition sont rincés soigneusement, généralement à l'eau, de préférence lavées au shampooing, rincées puis séchées ou laissées à sécher.

Selon un deuxième mode de réalisation particulier, l'invention a pour objet un procédé de décoloration des fibres kératiniques consistant à mettre en contact la composition prête à l'emploi, avec lesdites fibres pendant une durée suffisante pour obtenir l'effet souhaité.

Conformément à une première variante de ce deuxième mode de réalisation, la composition prête à l'emploi comprend, outre le ou les composés portant les quatre fonctions carboxylique et le ou les polymères épaississants, au moins un agent oxydant.

Une première possibilité est constituée par un procédé mis en oeuvre à un pH acide (soit inférieur à 7). De préférence, l'agent oxydant est le peroxyde d'hydrogène.

Selon cette première possibilité, la composition appliquée sur les fibres kératiniques comprend au moins un agent oxydant, le composé et le polymère épaississant.

Une deuxième possibilité est constituée par un procédé mis en oeuvre à un pH basique (soit supérieur ou égal à 7).

Dans ce cas, la composition prête à l'emploi appliquée sur les fibres est de préférence obtenue par mélange extemporané, avant l'application sur les fibres, d'une première composition comprenant au moins un persel, avec une deuxième composition aqueuse comprenant un agent oxydant, et éventuellement une troisième composition comprenant au moins un agent alcalin ; le(s) polymère(s) épaississant(s) et le composé comportant quatre fonctions carboxyliques sous forme acide ou salifiée, se trouvant, ensemble ou non, dans l'une et/ou l'autre des compositions précitées.

Classiquement, la première composition se présente sous une forme anhydre, et comprend au moins un persel, éventuellement au moins un agent alcalin, et éventuellement au moins un liquide inerte.

La deuxième composition, qui est une composition aqueuse, comprend de préférence à titre d'agent oxydant, le peroxyde d'hydrogène. Avantageusement, e pH de cette composition est inférieur ou égal à 7.

Comme indiqué auparavant, on peut éventuellement mettre en oeuvre une troisième composition, qui se présente aussi sous une forme aqueuse, comprenant au moins un agent alcalin.

Les listes données des divers ingrédients que l'on peut utiliser dans la composition selon l'invention restent valables et l'on pourra s'y reporter.

La composition prête à l'emploi est donc appliquée sur les fibres kératiniques, sèches ou humides, puis laissée pendant un temps de pose suffisant pour obtenir la décoloration recherchée. A l'issue de cette période, la composition est éliminée par un rinçage à l'eau, suivi d'un lavage avec un shampooing, puis éventuellement d'un séchage.

Habituellement, le temps de pose est compris entre quelques secondes et 30 minutes, de préférence entre 3 et 15 minutes.

La température à laquelle la composition est laissée agir est en général comprise entre la température ambiante (15 à 25°C) et 220°C, plus particulièrement entre la température ambiante et 80°C, de préférence entre 15 et 40 °C.

Conformément à une seconde variante de ce deuxième mode de réalisation, la composition prête à l'emploi comprend au moins un agent réducteur.

Cette variante est tout particulièrement appropriée lorsque l'on souhaite retirer une coloration artificielle, dans la mesure où celle-ci peut être éliminée en mettant en oeuvre une réaction de réduction. On parle alors de décapage.

Dans ce cas, la composition prête à l'emploi est appliquée sur des fibres teintes au moyen de colorants d'oxydation (base et/ou coupleur), de colorants directs, ou encore d'une combinaison de ces deux familles de colorants.

Dans le cas plus particulier où la composition prête à l'emploi comprend à titre d'agent réducteur, au moins une réductone ou dérivé d'acide sulfinique, le pH de la composition prête à l'emploi est de manière avantageuse inférieure à 7, de préférence compris entre 3 et 7. Le pH de la composition peut être adapté de manière classique en utilisant, selon les besoins, un ou plusieurs agents alcalinisants ou acidifiants, tels que notamment ceux listés plus haut.

Le procédé consiste donc à appliquer sur les fibres kératiniques la composition prête à l'emploi puis à laisser poser la composition pendant un temps suffisant pour obtenir la décoloration, à rincer les fibres pour en éliminer la composition, à les laver avec un shampooing, à les rincer et éventuellement les sécher.

Habituellement, le temps de pose varie entre quelques secondes et 60 minutes, de préférence entre 1 et 60 minutes, et plus préférentiellement entre 10 et 50 minutes.

La température à laquelle la composition est laissée agir est en général comprise entre la température ambiante (15 à 25°C) et 220°C, plus spécialement entre la température ambiante et 80°C, et de préférence entre 15 et 40 °C.

Selon un troisième mode de réalisation particulier, l'invention a pour objet un procédé de coloration des fibres kératiniques humaines dans lequel on met en contact la composition prête à l'emploi avec lesdites fibres pendant une durée suffisante pour obtenir l'effet souhaité.

Selon une première possibilité, la composition prête à l'emploi ne comprend pas d'agent oxydant, elle peut être appliquée directement sur les fibres.

Dans ce cas, la composition prête à l'emploi comprend, outre le(s) composé(s) portant quatre fonctions carboxyliques, le(s) polymère(s) épaississant(s), au moins un colorant direct. Cette composition peut être stockée telle quelle ou bien résulter du mélange extemporané d'au moins une composition comprenant au moins un polymère épaississant, d'au moins une composition comprenant au moins un colorant direct : le composé portant quatre fonctions carboxyliques pouvant se trouver dans l'une et/ou l'autre des compositions. De manière avantageuse, la composition prête à l'emploi peut être stockée telle quelle.

Dans le cas de cette première possibilité de procédé, on applique la composition prête à l'emploi pendant le temps nécessaire au développement de la coloration, puis à laisser sécher ou à sécher les fibres, sans rinçage final ou bien avec un rinçage.

On peut aussi envisager avec ou sans rinçage en fin de temps de pose, d'effectuer un lavage avec un shampooing des fibres, de les rincer et de les sécher ou les laisser sécher.

Le temps de pose est d'environ quelques secondes à 60 minutes, plus particulièrement de 5 à 60 minutes et de préférence 5 à 40 minutes.

La température nécessaire au développement de la coloration est généralement comprise entre la température ambiante (15 à 25°C) et 220°C, plus spécialement entre la température ambiante et 80°C, et avantageusement entre 15 et 40°C.

Selon une deuxième possibilité, la composition prête à l'emploi comprend au moins un agent oxydant. Comme indiqué auparavant, elle est de préférence obtenue par mélange extemporané, avant l'application, d'au moins une composition comprenant au moins un polymère épaississant, au moins une base d'oxydation et/ou au moins un coupleur et/ou au moins un colorant direct avec au moins une composition oxydante ; le composé portant quatre fonctions acide carboxylique se trouvant dans l'une ou plusieurs des compositions précitées.

La composition oxydante employée dans ce cas est classique et correspond habituellement à une composition aqueuse comprenant un agent oxydant tel que listé auparavant.

La composition oxydante peut de même comprendre les additifs usuels dans le domaine, comme par exemple des agents tensioactifs, des liquides hydrophobes (si la composition est sous forme émulsionnée) ; des agents séquestrants ; des agents stabilisants le peroxyde d'hydrogène ; des agents anti-mousse, etc.

Comme précédemment, le temps de pose est d'environ quelques secondes à 60 minutes, plus particulièrement de 5 à 60 minutes et de préférence 5 à 40 minutes.

Concernant la température nécessaire au développement de la coloration, celle-ci est généralement comprise entre la température ambiante (15 à 25°C) et 220°C, plus spécialement entre la température ambiante et 80°C, et avantageusement entre 15 et 40°C.

Une fois la coloration souhaitée obtenue, après éventuellement un rinçage des fibres, on effectue habituellement un lavage des fibres avec un shampooing, on les rince et on les sèche ou on les laisse sécher.

Les exemples qui suivent illustrent la présente invention sans toutefois en limiter la portée.

### EXEMPLE 1

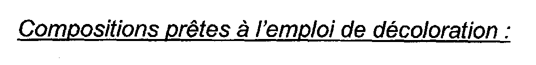

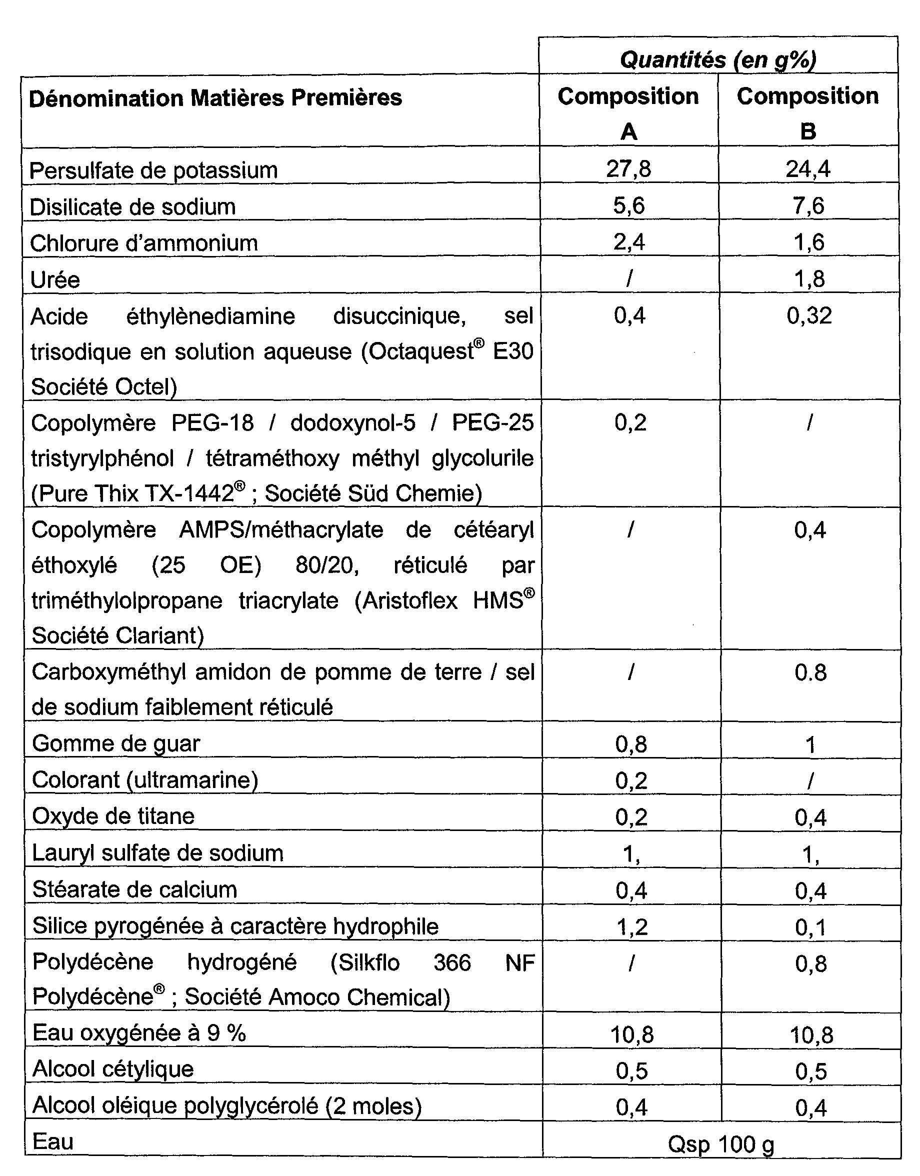

On applique la composition A pendant 45 minutes sous casque, sur cheveux naturels foncés, puis on rince abondamment à l'eau.

La composition A présente une texture idéale pour une application facile et rapide, qui ne coule et n'évolue pas pendant le temps de pose.

On obtient une décoloration puissante et homogène.

On applique la composition B pendant 30 minutes sous casque sur cheveux naturels foncés, puis on rince abondamment à l'eau.

La composition A présente une texture idéale pour une application facile et rapide, qui ne coule et n'évolue pas pendant le temps de pose.
On obtient une décoloration puissante et homogène, et les cheveux sont doux, brillants et faciles à démêler.

### EXEMPLE 2

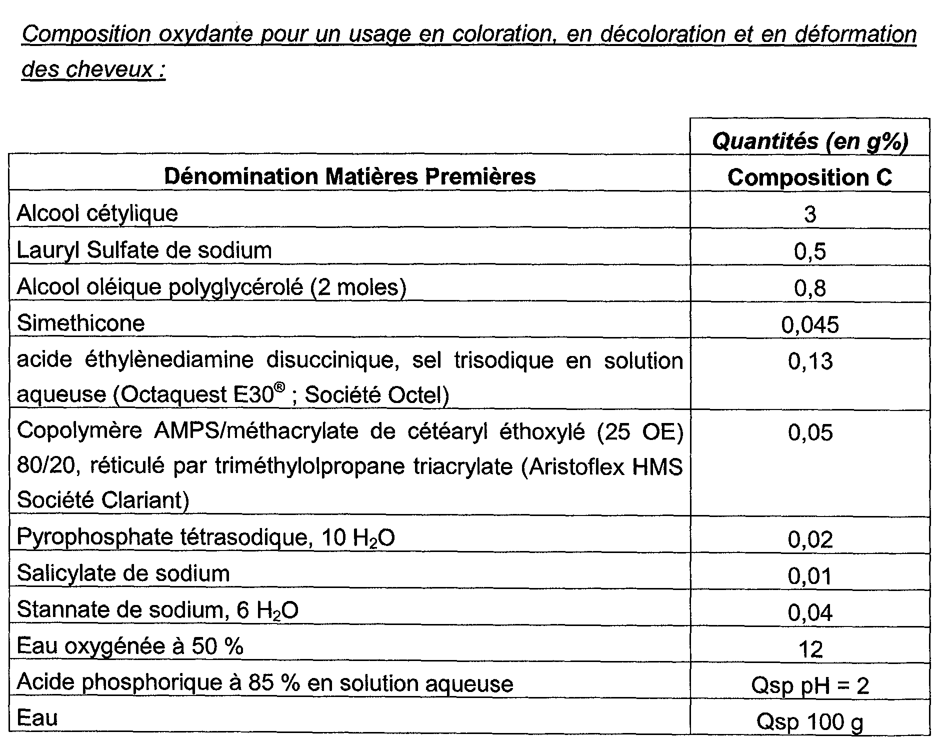

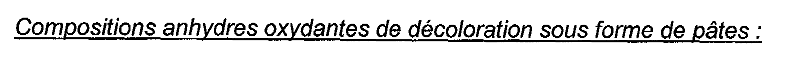

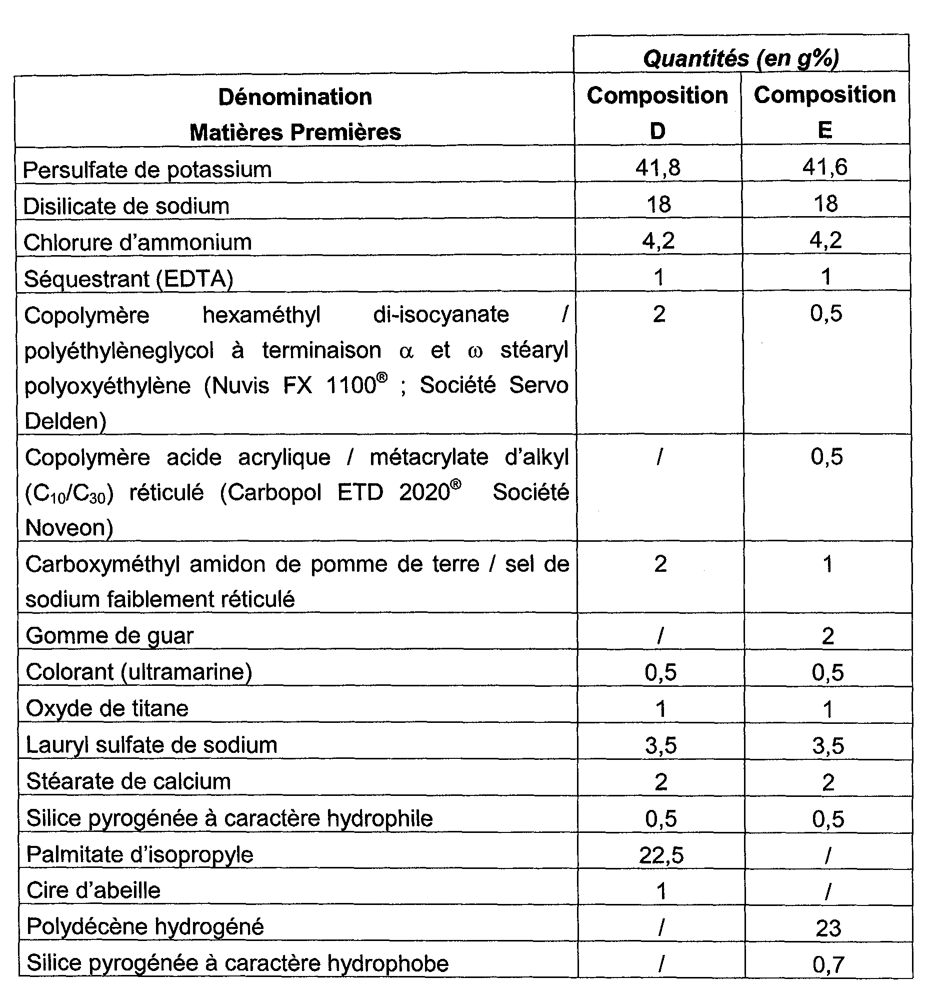

On mélange 40 g de la composition de décoloration D avec 80 g de la composition oxydante à base d'eau oxygénée C. On applique le mélange décolorant prêt à l'emploi ainsi obtenu 45 minutes sous casque sur cheveux naturels foncés, puis on rince abondamment à l'eau.

On mélange 40 g de la composition de décoloration E avec 60 g de la composition oxydante à base d'eau oxygénée C. On applique le mélange décolorant prêt à l'emploi ainsi obtenu 25 minutes sous casque sur cheveux naturels foncés, puis on rince abondamment à l'eau.

Dans chacun des deux cas, la composition prête à l'emploi obtenue présente une texture idéale pour une application facile et rapide, ne coule et n'évolue pas pendant le temps de pose.

On obtient une décoloration puissante et homogène, avec des cheveux doux, brillants et faciles à démêler.

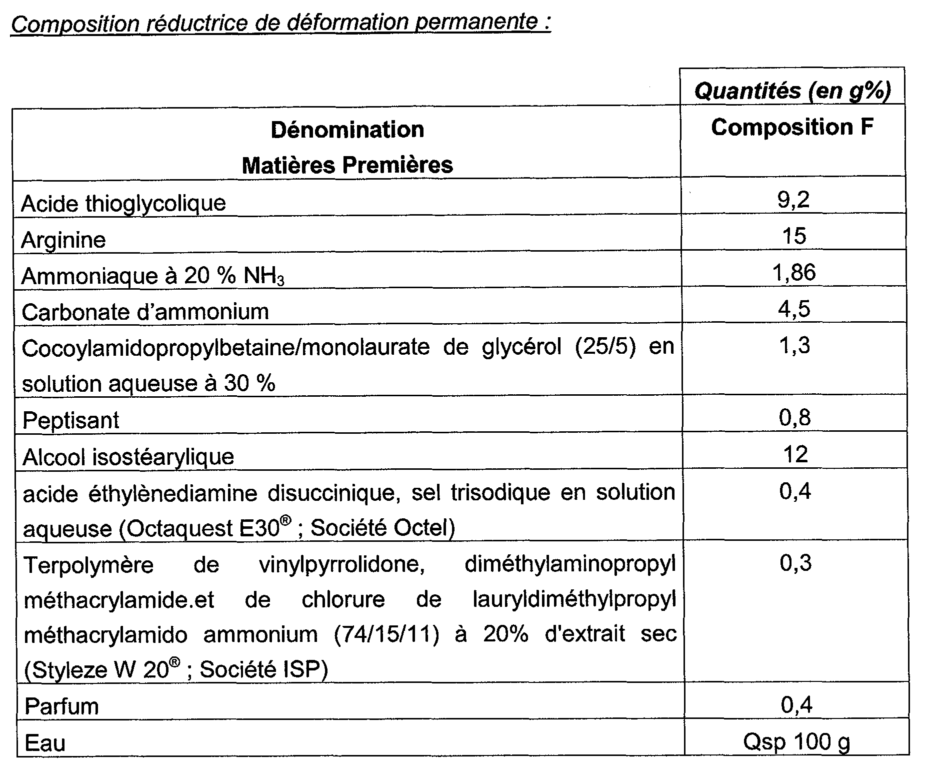

La composition réductrice F est appliquée sur une mèche de cheveux humides, préalablement enroulée sur un bigoudi de 9 mm de diamètre. Le temps de pose est de 10 minutes.

La composition présente une texture idéale pour une application facile et rapide, qui ne coule pas et n'évolue pas pendant le temps de pose

Une fois la temps écoulé, on rince abondamment la mèche à l'eau, puis on applique ensuite la composition oxydante C. Le temps de pose est de 10 minutes.

On rince ensuite la mèche obtenue à l'eau et l'on déroule les cheveux du bigoudi, puis on sèche.

On obtient une mèche ondulée.

### EXEMPLE 3

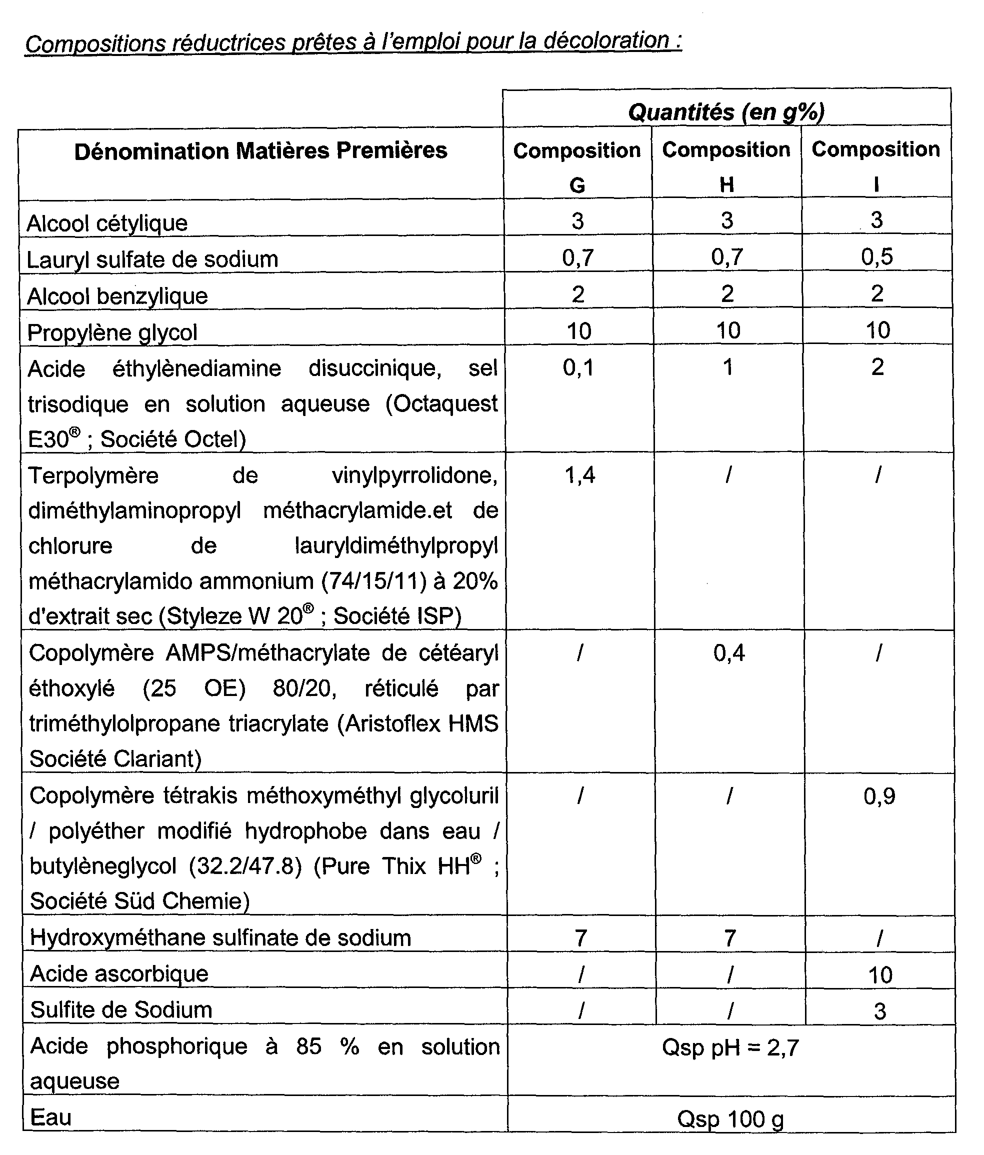

Les compositions de décoloration G, H et I sont appliquées sur des cheveux gris naturels à 90% de blancs, préalablement colorés par la nuance 6.66 de la gamme L'OREAL MAJIROUGE®, avec un rapport de bain de 10 g de produit pour 1 g de cheveux.

Après 30 minutes de pose, les mèches sont rincées, puis traitées 2 minutes à l'aide d'une solution aqueuse de peroxyde d'hydrogène à 3 %, puis rincées à nouveau.

On réalise ensuite un shampooing standard, puis on procède au séchage des cheveux.

On obtient une décoloration puissante et homogène. Le reflet rouge intense a pratiquement disparu, laissant apparaître à nouveau les cheveux substantiellement tels qu'ils étaient avant l'application de la teinture.

### EXEMPLE 4

### Compositions de coloration directe :

On a préparé les compositions suivantes (exprimées en grammes) :

### Composition oxydante :

| | |
|---|---|
| Alcool gras | 2,3 |
| Alcool gras oxyéthyléné | 0,6 |
| Amide gras | 0,9 |
| Glycérine | 0,5 |
| Peroxyde d'hydrogène | 7,5 |
| parfum | qs |
| Eau déminéralisée qsp | 100 |

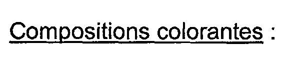

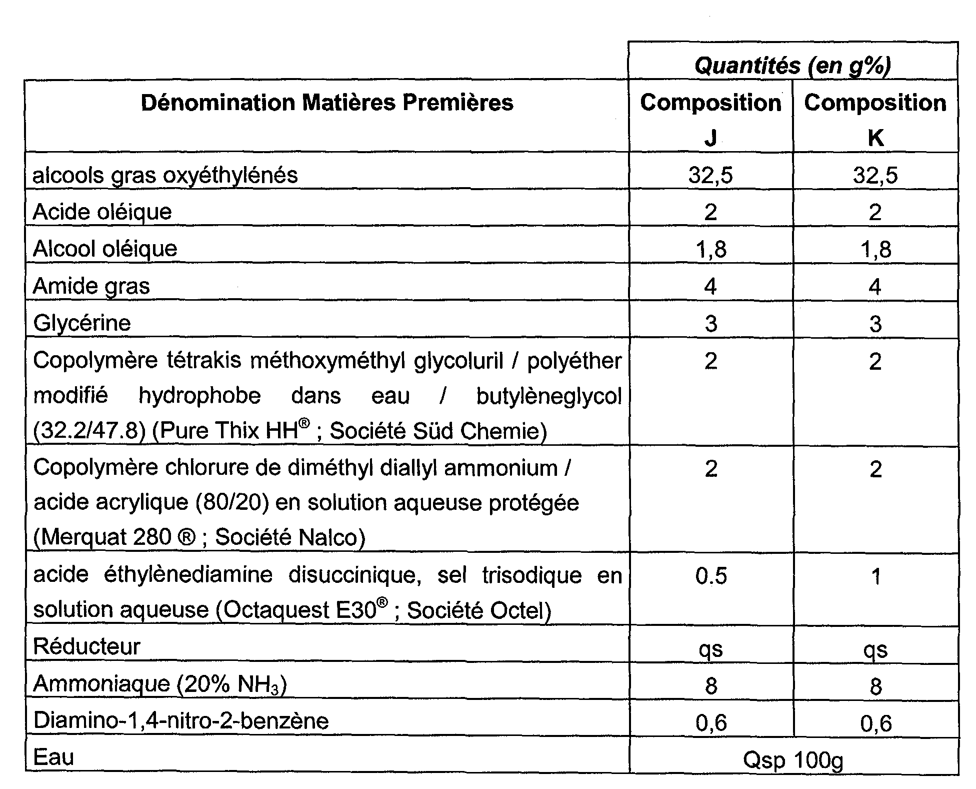

Les compositions colorantes ont été mélangées, au moment de l'emploi, dans un bol en plastique et pendant 2 minutes, à la composition oxydante donnée ci-dessus, à raison de 1 partie de composition colorante pour 1,5 parties de composition oxydante.

On a appliqué les mélanges obtenus sur des mèches de cheveux naturels à 90% de blancs et on a laissé poser 30 minutes.

Les compositions prêtes à l'emploi ainsi obtenues présentent une texture idéale pour une application facile et rapide, qui ne coule pas et n'évolue pas pendant le temps de pose.

On a ensuite rincé les mèches à l'eau, on les a lavées au shampooing standard, à nouveau rincées à l'eau, puis séchées et démêlées.

Les cheveux ont été teints dans les deux cas dans une nuance rouge puissante, et les cheveux ne sont pas rêches.

### EXEMPLE 5

On a préparé la composition L suivante (exprimée en grammes) :

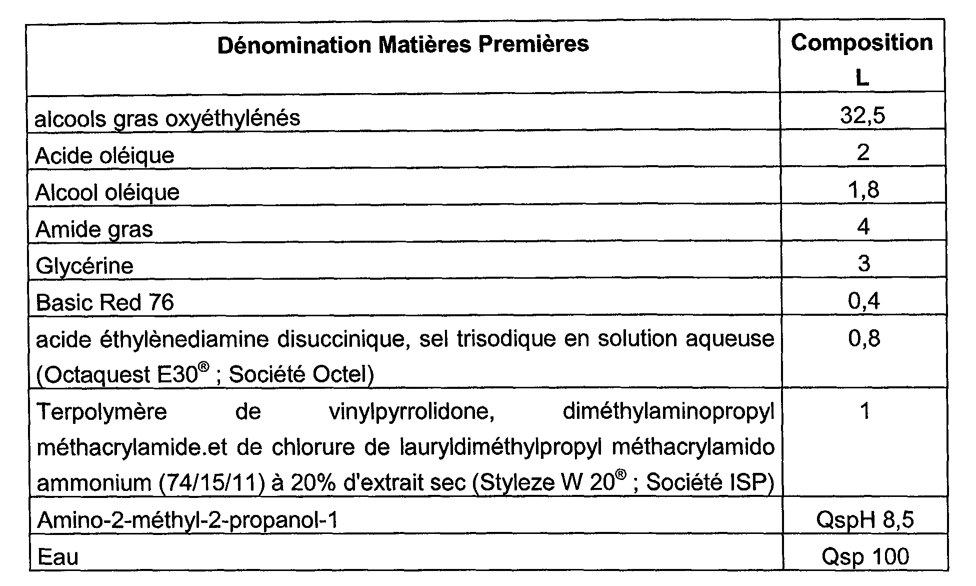

Cette composition a été appliquée 30minutes sur des cheveux gris à 90% de blancs. Après rinçage et séchage les cheveux ont été colorés en rouge intense et les cheveux ne sont pas rêches.

### EXEMPLE 6

### Compositions de coloration d'oxydation :

On a préparé les compositions colorantes suivantes :

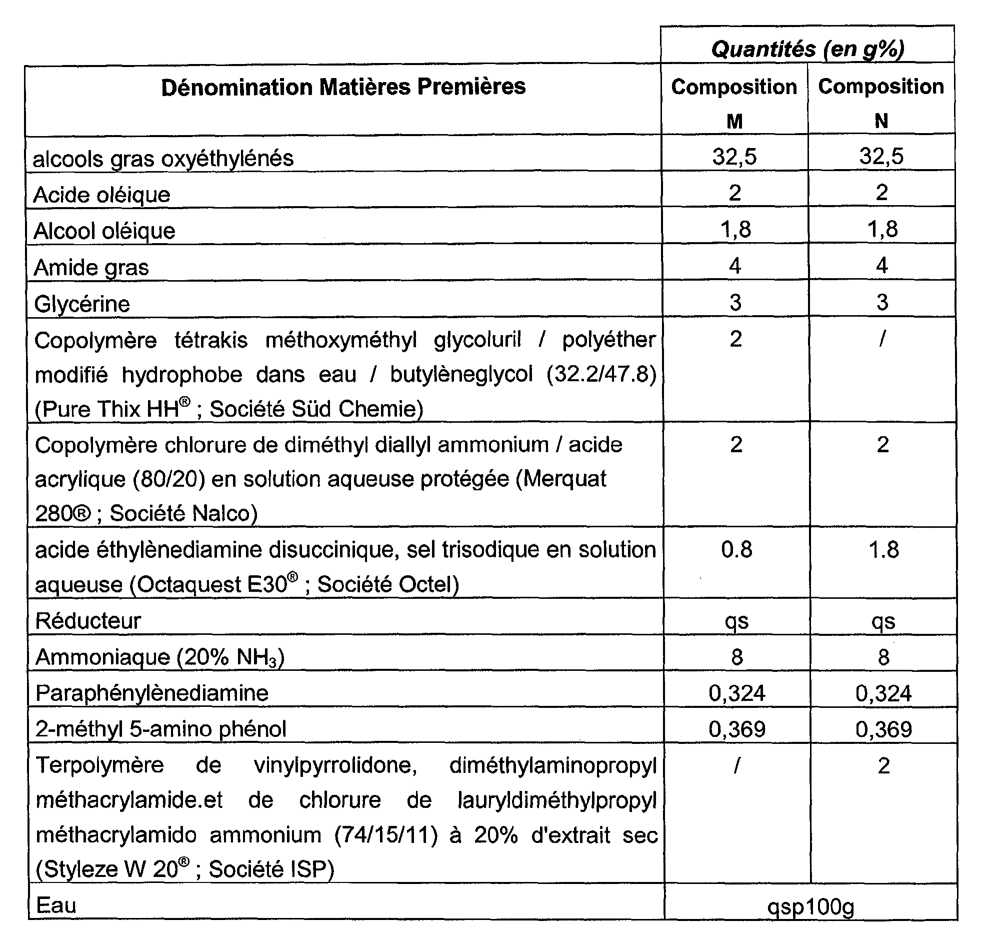

Les compositions colorantes ont été mélangées, au moment de l'emploi, dans un bol en plastique et pendant 2 minutes, à la composition oxydante C, à raison de 1 partie de composition colorante pour 1,5 parties de composition oxydante.

On a appliqué les mélanges obtenus sur des mèches de cheveux naturels à 90% de blancs et on a laissé poser 30 minutes.

Les compositions prêtes à l'emploi ainsi obtenues présentent une texture idéale pour une application facile et rapide, qui ne coule pas et n'évolue pas pendant le temps de pose.

On a ensuite rincé les mèches à l'eau, on les a lavées au shampooing standard, à nouveau rincées à l'eau, puis séchées et démêlées.

Les cheveux ont été teints dans les deux cas dans une nuance pourpre-rouge puissante, et les cheveux ne sont pas rêches.

## Revendications

1. Composition destinée au traitement de fibres kératiniques humaines, comprenant :
- au moins un composé choisi parmi l'acide éthylènediamine-N,N'-disuccinique, un sel, ou leur mélange,
- au moins un polymère épaississant choisi parmi les polymères à squelette aminoplaste-éther, les polymères cationiques associatifs, les homopolymères réticulés d'acide 2-acrylamido-2-méthylpropane sulfonique, ou les copolymères associatifs comprenant au moins un monomère à groupement sulfonique.

2. Composition selon l'une quelconque des revendications précédentes, **caractérisée en ce que** la teneur en composé est comprise entre 0,01 et 20 % en poids par rapport au poids de la composition, plus particulièrement entre 0,1 et 10 % en poids par rapport au poids de la composition, de préférence entre 0,2 et 5 % en poids par rapport au poids de la composition.

3. Composition selon l'une quelconque des revendications précédentes, **caractérisée en ce que** les polymères cationiques associatifs sont choisis parmi les polyuréthanes cationiques amphiphiles, les polyvinyllactames cationiques amphiphiles, les dérivés de cellulose cationiques amphiphiles, ou leurs mélanges.

4. Composition selon l'une quelconque des revendications précédentes, **caractérisée en ce que** les copolymères associatifs comprenant au moins un monomère à groupement sulfonique sont choisis parmi les copolymères associatifs comprenant au moins un monomère du type de l'acide vinylsulfonique, l'acide styrènesulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique, seuls ou en mélange.

5. Composition selon l'une quelconque des revendications précédentes, **caractérisée en ce que** la teneur en polymère épaississant représente 0,01 à 20 % en poids par rapport au poids de la composition, de préférence entre 0,1 et 10 % en poids par rapport au poids de la composition, et de manière encore plus avantageuse, entre 0,5 et 5 % en poids par rapport au poids de la composition.

6. Composition selon l'une quelconque des revendications précédentes, **caractérisée en ce qu'**elle comprend au moins un agent oxydant.

7. Composition selon la revendication précédente, **caractérisée en ce que** l'agent oxydant est choisi parmi le peroxyde d'hydrogène, le peroxyde d'urée, les bromates ou ferricyanures de métaux alcalins, les persels tels que les perborates et les persulfates, seuls ou en mélanges.

8. Composition selon l'une quelconque des revendications précédentes, **caractérisée en ce qu'**elle se présente sous la forme d'une pâte anhydre.

9. Composition selon l'une quelconque des revendications 1 à 4, **caractérisée en ce qu'**elle comprend au moins un agent réducteur.

10. Composition selon la revendication précédente, **caractérisée en ce que** l'agent réducteur est choisi parmi les réductones, les composés comprenant du soufre, et notamment des composés présentant au moins une fonction thiol, (di)sulfure, sulfite, sulfinique, sous forme de sel ou non.

11. Composition selon l'une quelconque des revendications 1 à 8, **caractérisée en ce qu'**elle comprend au moins un colorant direct, au moins une base d'oxydation éventuellement associée à au moins un coupleur, ou leurs mélanges.

12. Composition selon la revendication précédente, **caractérisée en ce que** le colorant direct est un colorant cationique ou non ionique, choisi parmi les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane, seuls ou en mélanges.

13. Composition selon la revendication 11, **caractérisée en ce que** la base d'oxydation est choisie parmi les o-phénylènediamines, les p-phénylènediamines, les bis-phénylènediamines, les o-aminophénols, les p-aminophénols, les bis-para-aminophénols, les bases hétérocycliques, leurs sels d'addition avec un acide, ainsi que leurs mélanges.

14. Composition selon la revendication 11, **caractérisée en ce que** le coupleur est choisi parmi les m-aminophénols, les m-phénylènediamines, les m-diphénols, les naphtols, les coupleurs hétérocycliques, leurs sels d'addition avec un acide, ainsi que leurs mélanges.

15. Procédé de déformation permanente des fibres kératiniques humaines consistant à mettre en contact une composition selon l'une quelconque des revendications 1 à 10, avec lesdites fibres pendant une durée suffisante pour obtenir l'effet souhaité.

16. Procédé de décoloration des fibres kératiniques humaines consistant à mettre en contact une composition selon l'une quelconque des revendications 1 à 10, avec lesdites fibres pendant une durée suffisante pour obtenir l'effet souhaité.

17. Procédé de coloration des fibres kératiniques humaines consistant à mettre en contact une composition selon l'une quelconque des revendications 1 à 7 et 12 à 14, avec lesdites fibres pendant une durée suffisante pour obtenir l'effet souhaité.
